# EUROPÄISCHE PATENTSCHRIFT

(11) **EP 2 234 614 B1**
(45) Veröffentlichungstag und Bekanntmachung des Hinweises auf die Patenterteilung: **17.10.2012**
(21) Anmeldenummer: 08863830.9
(22) Anmeldetag: 22.12.2008
(51) Int. Cl.: A61K 31/24, A61K 31/5377, A61K 31/724, A61K 47/48, A61K 47/40, A61K 9/00, A61P 9/00, B82Y 5/00

(54) **PHARMAZEUTISCHE ZUSAMMENSETZUNG ZUR PARENTERALEN VERABREICHUNG EINES ULTRAKURZWIRKSAMEN BETA-ADRENOREZEPTOR ANTAGONISTEN**
PHARMACEUTICAL COMPOSITION FOR PARENTERAL ADMINISTRATION OF AN ULTRASHORT ACTING BETA-ADRENORECEPTOR ANTAGONIST
COMPOSITION PHARMACEUTIQUE POUR L'ADMINISTRATION PARENTÉRALE D'UN ANTAGONISTE DE BÊTA-ADRÉNORÉCEPTEUR À ACTIVITÉ ULTRARAPIDE

(30) Priorität: 21.12.2007 AT 21072007
(43) Veröffentlichungstag der Anmeldung: 06.10.2010
(73) Patentinhaber: AOP Orphan Pharmaceuticals AG, 1160 Wien (AT)
(72) Erfinder: WIDMANN, Rudolf Stefan, A-3002 Purkersdorf (AT)
(74) Vertreter: Loidl, Manuela Bettina
(86) Internationale Anmeldenummer: PCT/AT2008/000470
(87) Internationale Veröffentlichungsnummer: WO 2009/079679

(56) Entgegenhaltungen:
- WO-A-85/04580
- WO-A-94/12217
- WO-A-02/076446
- WO-A-03/028718
- WO-A-2007/012974
- CN-A- 1 827 109
- PITHA J ET AL: "Drug solubilizers to aid pharmacologists: Amorphous cyclodextrin derivatives" LIFE SCIENCES, PERGAMON PRESS, OXFORD, GB, Bd. 43, Nr. 6, 1. Januar 1988 (1988-01-01), Seiten 493-502, XP023720898 ISSN: 0024-3205 [gefunden am 1988-01-01]
- RAJEWSKI R A ET AL: "PHARMACEUTICAL APPLICATIONS OF CYCLODEXTRINS. 2. IN VIVO DRUG DELIVERY" JOURNAL OF PHARMACEUTICAL SCIENCES, AMERICAN PHARMACEUTICAL ASSOCIATION, WASHINGTON, US, Bd. 85, Nr. 11, 1. November 1996 (1996-11-01), Seiten 1142-1169, XP000629515 ISSN: 0022-3549
- LOFTSSON THORSTEINN ET AL: "CYCLODEXTRINS IN DRUG DELIVERY" EXPERT OPINION ON DRUG DELIVERY, ASHLEY PUBLICATIONS, GB, Bd. 2, Nr. 2, 1. März 2005 (2005-03-01), Seiten 335-351, XP008075998 ISSN: 1742-5247
- REN X ET AL: "SEPARATION OF CHIRAL BASIC DRUGS WITH SULFOBUTYL-BETA-CYCLODEXTRIN IN CAPILLARY ELECTROPHORESIS" CHROMATOGRAPHIA, WIESBADEN, DE, Bd. 50, Nr. 5/06, 1. September 1999 (1999-09-01), Seiten 363-368, XP008061808 ISSN: 0009-5893

## Beschreibung

Die vorliegende Erfindung betrifft eine pharmazeutische Zusammensetzung zur parenteralen Verabreichung eines ultrakurzwirksamen β-Adrenorezeptor Antagonisten in Form einer Lösunggemäß der Ansprüche und die Verwendung eines Cyclodextrins und/oder eines funktionellen Cyclodextrin-Derivates zur Erhöhung der Stabilität eines ultrakurzwirksamen β-Adrenorezeptor Antagonisten in einer zur parenteralen Verabreichung geeigneten lagerstabilen wässerigen Lösung gemäß der Ansprüche.

Ultrakurzwirksame β-Adrenorezeptor Antagonisten wie Esmolol-Hydrochlorid, Landiolol-Hydrochlorid (zwei cardio-selektive β₁-Blocker), und Flestolol - Hydrochlorid werden aufgrund ihrer sehr kurzen Eliminationshalbwertszeit in Form von parenteralen Formulierungen v.a. in der Anästhesie sowie in der Notfall- und Intensivmedizin eingesetzt.

Als "ultrakurawirksam" wird für die Zwecke der vorliegenden Erfindung ein Wirkstoff verstanden, dessen Eliminationshalbwertszeit geringer ist als jene des β₁-Blockers Metoprolol (Halbwertszeit t_{1/2} = 90 Minuten). Insbesondere beträgt die Eliminationshalbwertszeit der erfindungsgemäß eingesetzten β-Adrenorezeptor Antagonisten bevorzugt weniger als 20 Minuten.

Ein Problem dabei ist einerseits die geringe Stabilität dieser Wirkstoffe in wässrigen Lösungen, da diese hydrolytisch zur freien Säure und zu Alkohol (im Fall von Esmolol zur reinen Säure und zu Methanol) gespalten werden [z.B.: Baaske DM, Dykstra SD, Wagenknecht DM, Karnatz NN Stability of esmolol hydrochloride in intravenous solutions. Am J Hosp Pharm. 1994 Nov 1;51(21):2693-6.; Tamotsu Yasuda, Hiroyuki Kamiya, Yoko Tanaka, Go Watanabe, Ultra-short-acting cardioselective beta-blockade attenuates postischemic cardiac dysfunction in the isolated rat heart, Eur J Cardiothorac Surg 2001; 19:647-652].

Andererseits ist die zur Verabreichung einzusetzende Wirkstoffkonzentration zum Beispiel an Esmolol so hoch, dass diese Lösungen oft hyperton sind. Zudem wird zur Stabilisierung dieser Lösungen in vielen Fällen auch Alkohol in Konzentrationen von um die 25% zugesetzt. Aufgrund dieser formulierungstechnischen Probleme ist die Anwendung von Esmolol in der Intensivmedizin mit zusätzlichen Risken verbunden.

Kommerziell erhältliches Landiolol hingegen ist zum Beispiel derzeit überhaupt nur in festen Formulierungen verfügbar, die vor ihrem Einsatz in der Intensivmedizin erst aufgelöst werden müssen, was zu unnötigen Zeitverlusten führen kann. Gefriergetrocknete Zusammensetzungen enthaltend Landiolol sind aus der CN 1827109A bekannt.

Versuche, die für Esmolol genannten Probleme zu lösen, umfassen den Zusatz von Dextrose [Wiest DB, Garner SS, Childress LM., Stability of esmolol hydrochloride in 5% dextrose injection. Am J Health Syst Pharm. 1995 Apr 1;52(7):716-8.], die Einstellung des pH-Wertes auf < 6 [Rosenberg LS, Hostetler CK, Wagenknecht DM, Aunet DA., An accurate prediction of the pH change due to degradation: correction for a "produced" secondary buffering system. Pharm Res. 1988 Aug;5(8):514-7.] sowie den Zusatz von Propylenglykol in hohen Konzentrationen [http://www.rxlist.com/cgi/generic3/esmolol.htm].

Die WO 85/04580 beschreibt Lösungen von Esmolol in einem Gewichtsanteil von 0,1% bis 30%, welche unter anderem einen Puffer und Ethanol in einem Anteil von 5 bis 60% enthalten.

Die EP 0 403 578 B1 beansprucht in Anspruch 1 eine injizierbare, wässrige Zusammensetzung zur Behandlung von Herzzuständen, die eine effektive Menge Esmolol (Hydrochlorid) in einem Anteil von 1 mg bis 250 mg Esmolol/ml Lösung und 0,01 bis 0,04 M Puffer umfasst sowie einen pH-Wert im Bereich von 4,5 bis 5,5 aufweist.

Die Internationale Anmeldung WO 02/076446 beschreibt eine Esmolol-Lösung, die 0,1 bis 500 mg/ml Esmolol-Hydrochlorid, 0,01 bis 2 M Puffer und 1 bis 500 mg/ml eines Osmoseeinstellenden Mittels enthält.

Die Europäische Patentanmeldung EP 1 417 962 A beschreibt pharmazeutische Zusammensetzungen, die aus 30 ml bis 70 ml Verdünnung mit einem Anteil von 1500 mg bis 3500 mg Esmolol oder eines pharmazeutisch verträglichen Salzes davon bestehen.

Versuche, die für Landiolol genannten Probleme zu lösen, sind nicht bekannt.

Die Aufgabe der vorliegenden Erfindung besteht darin, eine Zusammensetzung zur parenteralen Verabreichung von ultrakurzwirksamen β-Adrenorezeptor Antagonisten, insbesondere zur Injektion bzw. Infusion, zur Verfügung zu stellen, welche einerseits eine hohe Lagerstabilität und andererseits eine für die Verabreichung geeignete Osmolarität aufweist. Die Zusammensetzung soll außerdem möglichst gefäßschonende Eigenschaften haben, was besonders bei hohen Wirkstoff-Konzentrationen (z.B. im Fall von Esmolol) wichtig ist.

Es wurde überraschenderweise gefunden, dass die Verwendung eines Cyclodextrins und/oder eines funktionellen Cyclodextrin-Derivates ausgewählt aus der Gruppe bestehenol aus α-Cyclodextrin, β-Cyclodextrin, α-Cyclodextrin, (2-Hydroxypropyl)-β-Cyclodextrin und (Sulfobutylether)-7β-cyclodextrin zur Erhöhung der Stabilität eines ultrakurzwirksamen β-Adrenorezeptor Antagonisten ausgewähtt aus der Gruppe, bestehend aus Esmolol, Landiclol, Flestolol und/oder eines pharmazeutisch akzeptablen Salzes davon in einer zur parenteralen Verabreichung geeigneten lagerstabilen wässerigen Lösung die Aufgabe der Erfindung in hervorragender Weise löst.

In diesem Aspekt der vorliegenden Erfindung kann die Lösung auch weitere Hilfsstoffe, insbesondere Puffer, Konservierungsmittel, mit Wasser mischbare organische Lösungsmittel, Salze, Zuckeralkohole und/oder Zucker enthalten.

In einem weiteren Aspekt betrifft die vorliegende Erfindung eine pharmazeutische Zusammensetzung zur parenteralen Verabreichung eines ultrakurzwirksamen β-Adrenorezeptor Antagonisten in Form einer lagerstabilen Lösung, im wesentlichen bestehend aus
a) einem ultrakurzwirksamen β-Adrenorezeptor Antagonisten ausgewähtt aus der Gruppe, bestehend aus Esmolol, Landiclol, Flestolol und/oder einem pharmazeutisch akzeptablen Salz davon
b) Wasser und
c) einem Cyclodextrin und/oder einem funktionellen Cyclodextrin-Derivat ausgewählt aus der Gruppe bestehenol aus α-Cyclodextrin, β-Cyclodextrin, α-Cyclodextrin, (2-Hydroxypropyl)-β-Cyclodextrin und (Sulfobutylether)-7β-cyclodextrin.

DE 42 07 922 A1 und US 2004/0053894 beschreiben allgemein die Verwendung von Cyclodextrin in pharmazeutischen Zusammensetzungen. Weiters wird die Verwendung von Cyclodextrin in pharmazeutischen Zusammensetzungen in der WO 2003/033025 A2, der US 2003/0216349 A1 und in Ikeda et al., J Pharm Sci. 2004, 93(7), 1659-1671, beschrieben.

Es wurde gefunden, dass durch Zusatz von Cyclodextrin und/oder einem funktionellen Cyclodextrin-Derivat die Stabilität von ultrakurzwirksamen β-Adrenorezeptor Antagonisten in rein wässriger Lösung entscheidend erhöht werden kann.

Überraschenderweise sind außerdem ultrakurzwirksame β-Adrenorezeptor Antagonisten-Lösungen, welche lediglich aus dem ultrakurzwirksamen β-Adrenorezeptor Antagonisten, Cyclodextrin und Wasser bestehen, auch ohne Vorhandensein weiterer Hilfstoffe (wie z.B. den aus dem Stand der Technik bekannten Puffern oder die Osmolarität einstellenden Mitteln) ausreichend lagerstabil und hinsichtlich der osmolaren Eigenschaften zur Verabreichung geeignet. Dies gilt insbesondere im pH-Bereich von 3 bis 7,5 bzw. besonders bevorzugt 5 bis 7.

Unter dem Begriff "lagerstabil" wird für die Zwecke der vorliegenden Erfindung eine wässerige Lösung verstanden, die im Unterschied zu einer Lösung, die aus einem gefriergetrockneten Produkten durch Auflösen desselben hergestellt wird, über einen längeren Zeitraum aufbewahrt werden kann, ohne dass dabei ein nennenswerter Abbau des enthaltenen Wirkstoffes eintritt. Als "lagerstabil" werden insbesondere wässerige Lösungen verstanden, bei denen nach einem Monat weniger als 5 % des Wirkstoffes abgebaut sind.

Der pH-Wert der Lösung beträgt, wie bereits weiter oben erwähnt, bevorzugt 3 bis 7,5, insbesondere bevorzugt 5 bis 7.

Die Konzentration des Cyclodextrins bzw. des Cyclodextrin-Derivates in der Lösung beträgt dabei bevorzugt 0, 1 % bis 20% (w/v), bevorzugt 0,25% bis 7% (w/v), besonders bevorzugt 0,5% bis 4%.

Das Cyclodextrin bzw. das funktionelle Cyclodextrin-Derivat ist aus der Gruppe bestehend aus α-Cyclodextrin, β-Cyclodextrin, γ-Cyclodextrin, funktionellen Derivaten davon und Mischungen daraus ausgewählt.

Unter "funktionellen Cyclodextrin-Derivaten" sind (2-Hydroxypropyl)-β-cyclodextrin und (Sulfobutylether)-7β-cyclodextrin zu verstehen.

Der in der erfindungsgemäßen Zusammensetzung verwendete ultrakurzwirksame β-Adrenorezeptor Antagonist ist ein Wirkstoff ausgewählt aus der Gruppe bestehend aus Esmolol, Landiolol und Flestolol.

Die Konzentration des ultrakurzwirksamen β-Adrenorezeptor Antagonisten bzw. des Salzes davon in der Lösung kann je nach verwendetem β-Adrenorezeptor Antagonisten 0,1% bis 30% betragen.

Bevorzugte Konzentrationen im Fall von Esmolol bzw. Esmololsalzen sind 1 bis 20%. Bevorzugte Konzentrationen im Fall von Landiolol bzw. Landiololsalzen sind 1 bis 20%. Bevorzugte Konzentrationen im Fall von Flestolol bzw. Flestololsalzen sind 0,1 bis 10%.

Die Lösung weist bevorzugt eine Osmolarität von 270 mosmol/l bis 310 mosmol/l, insbesondere bevorzugt 280 mosmol/l bis 300 mosmol/l auf. Dies entspricht einer isotonen Lösung.

Im Fall von Esmolol liegt die erfindungsgemäße Zusammensetzung bevorzugt in Form einer Verkaufseinheit ausgewählt aus der Gruppe bestehend aus
- 1 ml Lösung enthaltend 10-20 mg Esmolol oder ein Salz davon
- 2 ml Lösung enthaltend 10-100 mg Esmolol oder ein Salz davon
- 5 ml Lösung enthaltend 50-500 mg Esmolol oder ein Salz davon
- 10 ml Lösung enthaltend 50-5000 mg, insbesondere 2500 mg Esmolol oder ein Salz davon
- 50 ml Lösung enthaltend 50-5000 Esmolol oder ein Salz davon
- 100 ml Lösung enthaltend 50-5000 mg Esmolol oder ein Salz davon
- 250 ml Lösung enthaltend 50-5000 mg Esmolol oder ein Salz davon vor.

In herkömmlichen Produkten kann eine 10 ml Lösung enthaltend 100 mg Esmolol als "ready-to-use"-Produkt unmittelbar zur Injektion eingesetzt werden. Herkömmliche Zusammensetzungen mit höheren Anteilen an Esmolol (insbesondere 10 ml/2500 mg Esmolol) müssen vor der Verabreichung verdünnt werden. Aufgrund ihrer gefäßschonenden Eigenschaften können demgegenüber erfindungsgemäße Lösungen auch mit höheren Konzentrationen an Esmolol als "ready-to-use"-Produkte verwendet werden.

Im Fall von Landiolol bzw. Flestolol liegt die erfindungsgemäße Zusammensetzung bevorzugt in Form einer Verkaufseinheit ausgewählt aus der Gruppe bestehend aus
- 1 ml Lösung enthaltend 5-20 mg Landiolol bzw. Flestolol oder ein Salz davon
- 2 ml Lösung enthaltend 5-100 mg Landiolol bzw. Flestolol oder ein Salz davon
- 5 ml Lösung enthaltend 10-500 mg Landiolol bzw. Flestolol oder ein Salz davon
- 10 ml Lösung enthaltend 10-5000 mg Landiolol bzw. Flestolol oder ein Salz davon
- 25 ml Lösung enthaltend 25-2500 mg Landiolol bzw. Flestolol oder ein Salz davon
- 50 ml Lösung enthaltend 50-5000 mg Landiolol bzw. Flestolol oder ein Salz davon
- 100 ml Lösung enthaltend 50-5000 mg Landiolol bzw. Flestolol oder ein Salz davon
- 250 ml Lösung enthaltend 50-5000mg Landiolol bzw. Flestolol oder ein Salz davon vor.

In herkömmlichen, Landiolol enthaltenden Produkten sind 50 mg Landiolol als Trockensubstanz enthalten. Diese Produkte müssen vor der Injektion zuerst in Lösung gebracht werden.

In den erfindungsgemäßen Zusammensetzungen von Landiolol liegt das Landiolol hingegen schon stabil in Lösung vor. Ganz allgemein können aufgrund der gefäßschonenden Eigenschaften der erfindungsgemäßen Zusammensetzungen vergleichsweise höhere Konzentrationen des jeweiligen ultrakurzwirksamen β-Adrenorezeptor Antagonisten als in bisherigen Formulierungen verwendet werden.

Die Herstellung der erfindungsgemäßen Zusammensetzung kann in an sich bekannter Weise durch Mischen und anschließendem Auflösen der jeweiligen Bestandteile erfolgen.

Die erfindungsgemäße Zusammensetzung kann insbesondere zur Herstellung eines Medikamentes zur Senkung der Ventrikelfrequenz bei Patienten mit Vorhofflimmern, Vorhofflattern und bei Sinustachykardie, bei atrioventrikulärer und bei AV-Knoten-Tachykardie, bei tachykarden supra- und ventrikulären Arrhythmien, bei Tachykardie und/oder Bluthochdruck vor, während und nach Operationen sowie in anderen Notfällen, zur Prophylaxe und Therapie perioperativer Ischämien, zur Therapie instabiler Angina pectoris und des akuten Myokardinfarkts verwendet werden.

Im folgenden wird die Erfindung anhand von Ausführungsbeispielen und Figuren näher erläutert.

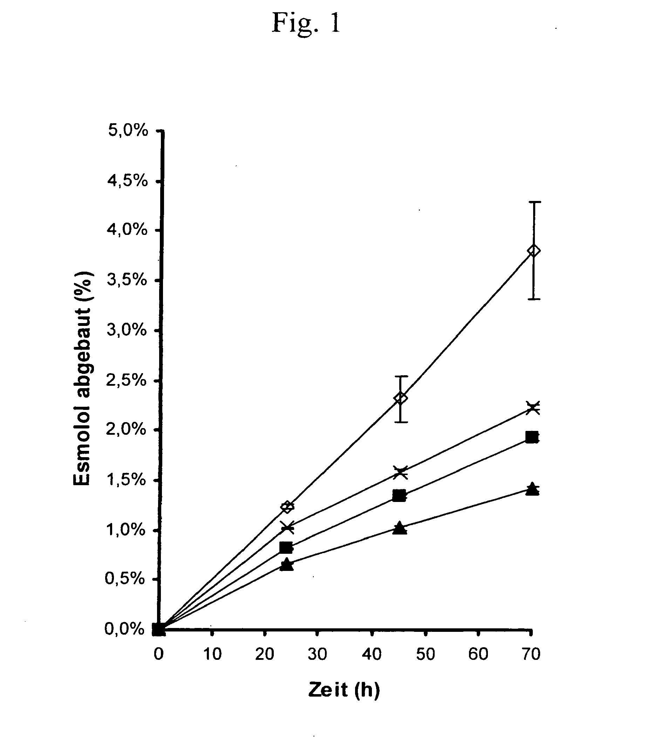

Dabei zeigt die Figur 1 den beschleunigten Abbau einer 5%-Esmolol-Referenzlösung und erfindungsgemäßen Zusammensetzungen bei 75°C.

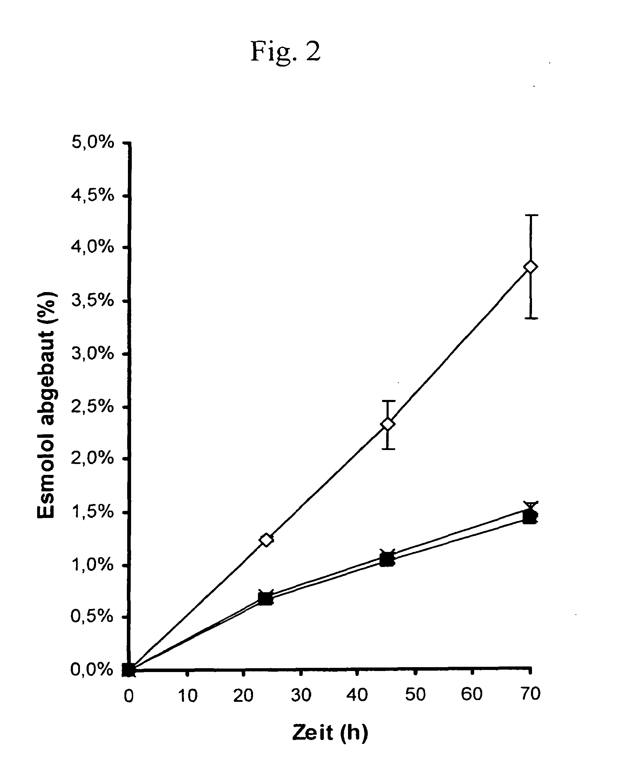

Figur 2 zeigt den Einfluss der Gefriertrocknung auf den beschleunigten Abbau von Esmolol-Cyclodextrin-Komplexen in Wasser bei 75°C.

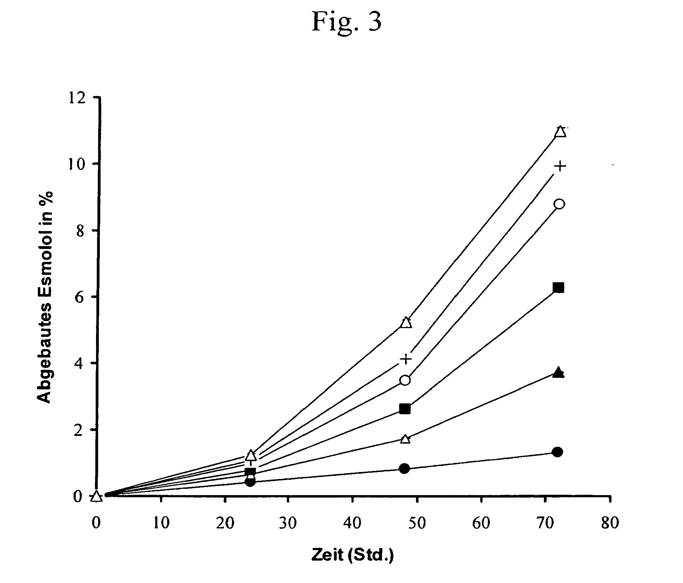

Desweiteren wird in Fig. 3 der Einfluss der Konzentration von α-Cyclodextrin auf die Stabilität von Esmolol bei 75°C gezeigt.

In Figur 4 wird der Einfluss von Hydroxypropyl-β-Cyclodextrin auf die Stabilität von Esmolol bei 75°C gezeigt.

Figur 5 zeigt den Einfluss von α-Cyclodextrin in ansteigenden Konzentrationen auf die Stabilität von Landiolol in wässriger Lösung bei 70°C.

Figur 6 zeigt den Einfluss von 2-Hydroxypropyl- β-Cyclodextrin in ansteigenden Konzentrationen auf die Stabilität von Landiolol in wässriger Lösung bei 70°C.

Figur 7 zeigt den Einfluss von γ-Cyclodextrin in ansteigenden Konzentrationen auf die Stabilität von Landiolol in wässriger Lösung bei 70°C.

Figur 8 zeigt den Einfluss des pH-Wertes auf die Stabilität von Landiolol in wässriger Lösung bei 70°C.

Figur 9 zeigt den Einfluss von Cyclodextrinen (2%; w/v), in die mittels konzentrierter Suspensionen Landiolol eingelagert wurde, auf die Stabilität wässriger Landiolol Lösungen von 0,25% (w/v) bei 70°C.

### BEISPIEL 1:

### Herstellung von (2-Hydroxypropyl-)β-cyclodextrin-Esmolol-Komplexen

Zu einer 5% igen Esmolol Lösung wurde eine äquimolare Menge an (2-Hydroxypropyl)-β-cyclodextrin gegeben und für 6 Stunden gerührt.

### BEISPIEL 2:

### Herstellung von α- und γ-Cyclodextrin-Esmolo-Komplexen

Zu einer 5%igen Esmolol-Lösung wurde jeweils eine äquimolare Menge an α-Cyclodextrin (Cavamax W6 Pharma, Hersteller Fa. Wacker Chemie AG) (Beispiel 2a) bzw. γ-Cyclodextrin (Cavamax W8 Pharma, Hersteller Fa. Wacker Chemie AG) (Beispiel 2b) gegeben und für 18 Stunden gerührt.

### BEISPIEL 3:

### Herstellung von Cyclodextrin-Esmolol-Komplexen

### Beispiel 3a:

Esmolol und α-Cyclodextrin werden in einer Endkonzentration von 5% (w/v) (Esmolol) bzw. 14% (w/v) (α-Cyclodextrin) in Wasser zu Injektions-Zwecken gelöst und für 24 Stunden bei Raumtemperatur gerührt.

### Beispiel 3b:

Esmolol und optional zusätzlich α-Cyclodextrin werden in einer Endkonzentration von 5% (w/v) (Esmolol) bzw. 14%, 7%, 4%, 2%, 1 % und 0% (w/v) (α-Cyclodextrin) in Wasser zu Injektions-Zwecken gelöst und für 24 Stunden bei Raumtemperatur gerührt.

### Beispiel 3c:

Esmolol und optional zusätzlich Hydroxypropyl-β-Cyclodextrin werden in einer Endkonzentration von 5% (w/v) (Esmolol) bzw. 7% und 0% (w/v) (Hydroxypropyl-β-Cyclodextrin) in Wasser zu Injektions-Zwecken gelöst und für 24 Stunden bei Raumtemperatur gerührt.

### BEISPIEL 4 - Vergleichsbeispiel:

### Herstellung einer State-of-the-Art 5% Esmolol Lösung zur Injektion

Gemäß der in Tabelle 1 aufgelisteten Rezeptur wurde eine parenterale Esmolol Lösung hergestellt.

**Tabelle 1 Zusammensetzung der parenteralen Esmolol Lösung**

| **Substanzen** | **Menge** |
|---|---|
| Esmolol HCl | 500 mg |
| Natrium-Acetat | 34 mg |
| Eisessig | 3,674 mg |
| Propylenglykol | 518 mg |
| Ethanol | 402 mg |
| HCl oder NaOH für pH 3,5 - 5,5 | q.s. |
| Wasser | ad 10 ml |

### BEISPIEL 5:

### Untersuchungen zur Stabilität von parenteralen Esmolol Lösungen

Mit den in den Beispielen 1 bis 3 beschriebenen Lösungen wurden beschleunigte Stabilitätstests bei einer Temperatur von 75°C durchgeführt. Nach 0, 24, 45 und 70 Stunden wurden Proben gezogen, die mit destilliertem Wasser verdünnt wurden (20 µl Probe + 180 µl Wasser). Der beschleunigte Abbau wurde mittels HPLC wie folgt bestimmt:

Für die qualitative und quantitative Analytik wurde ein HPLC-Gerät Hitachi Elite LaChrom mit Dioden-Array-Detektor und eine Waters Nova-Pak-Säule C18 4 µm 3,9x 150 mm verwendet. Die mobile Phase bestand aus (A) H₃PO₄ (10g/l) in Wasser, mit Triethylamin (TEA) auf pH 2,35 eingestellt und (B) Acetonitril. Der verwendete Gradient ist in Tabelle 2 aufgelistet.

**Tabelle 2 HPLC-Methode**

| Zeit (min) | A (%) | B (%) |
|---|---|---|
| 0 | 82 | 18 |
| 7 | 82 | 18 |
| 8 | 60 | 40 |
| 13 | 60 | 40 |
| 14 | 70 | 30 |
| 20 | 70 | 30 |
| Zeit (min) | A (%) | B (%) |
| 21 | 82 | 18 |
| 30 | 82 | 18 |

Die Flussrate betrug 1 ml/Minute, das Injektionsvolumen 20 µl. Die Detektion von Esmolol Hydrochlorid erfolgte bei 274 nm. Die Retentionszeit von Esmolol-Hydrochlorid betrug durchschnittlich 3,9 Minuten, die des Hauptabbauproduktes ("Verunreinigung A" in der Tabelle 3 unten) 1,7 Minuten. Zur Bestimmung des Abbaus wurde das Verhältnis des Hauptabbauproduktes zum verbleibenden Esmolol-Hydrochlorid berechnet und in Prozent angegeben ("Esmolol abgebaut (%)").

Die Ergebnisse dieser Studien zeigten eine entscheidend erhöhte Stabilität der Cyclodextrin enthaltenden Lösungen:

In Figur 1 wird der beschleunigte Abbau bei 75°C der 5% Esmolol Referenzlösung und Esmolol-Cyclodextrin-Komplexen in Wasser gezeigt [(◊) 5% Esmolol Vergleichslösung mit 0% Cyclodextrin- Beispiel 3b; (X) 5% Esmolol + γ-Cyclodextrin - Beispiel 2b); (■) 5% Esmolol + (2-Hydroxypropyl)-β-cyclodextrin - Beispiel 1); (▲) 5% Esmolol + α-Cyclodextrin - Beispiel 2a]. Die Werte sind Mittelwerte aus 3 Versuchen ± S.D.

In Figur 2 wird der Einfluss der Gefriertrocknung auf den beschleunigten Abbau von Esmolol-Cyclodextrin-Komplexen in Wasser bei 75°C gezeigt [(◊) 5% Esmolol Vergleichslösung mit 0% Cyclodextrin - Beispiel 3b; (X) 5% Esmolol in 14%iger α-Cyclodextrin-Lösung ohne Gefriertrocknung - Beispiel 3a; (■) 5% Esmolol + α-Cyclodextrin - Beispiel 2a mit anschließender Gefriertrocknung und Rekonstitution in Wasser]. Die Werte sind Mittelwerte aus 3 Versuchten ± S.D.

In Figur 3 wird der Einfluss der Konzentration von α-Cyclodextrin auf die Stabilität einer wässrigen 5% Esmolol Lösung bei 75°C gezeigt [(Δ) 5% Esmolol Vergleichslösung mit 0% α-Cyclodextrin - Beispiel 3b; (+) 5% Esmolol +1% α-Cyclodextrin - gemäß Beispiel 3b; (o) 5% Esmolol + 2% α-Cyclodextrin - gemäß Beispiel 3b; (■) 5% Esmolol + 4% α-Cyclodextrin - gemäß Beispiel 3b; (▲) 5% Esmolol + 7% α-Cyclodextrin - gemäß Beispiel 3b; (•) 5% Esmolol + 14% α-Cyclodextrin - gemäß Beispiel 3b;]. Die Werte sind Mittelwerte aus 3 Versuchten ± S.D.

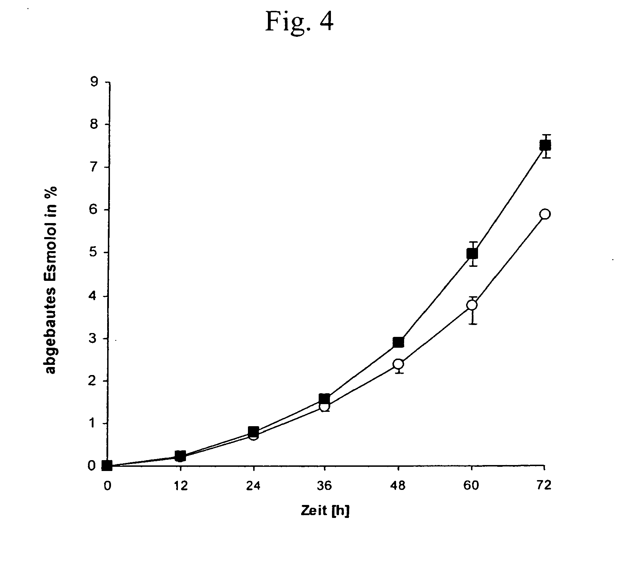

In Figur 4 wird der Einfluss von Hydroxypropyl-β-Cyclodextrin auf die Stabilität einer wässrigen 5% Esmolol Lösung bei 75°C gezeigt [(■) 5% Esmolol Vergleichslösung mit 0% Hydroxypropyl-β-Cyclodextrin - Beispiel 3c; (o) 5% Esmolol + 7% Hydroxypropyl-β-Cyclodextrin - gemäß Beispiel 3c]. Die Werte sind Mittelwerte aus 3 Versuchten ± S.D.

### BEISPIEL 6:

### Lagerungsstabilität von Esmolol-Cyclodextrin-Komplexen

Die Tabelle 3 zeigt die Lagerungsstabilität von Esmolol-Cyclodextrin-Komplexen (Beispiel 3a) im Vergleich zu einer State-of-the-art Formulierung (Beispiel 4) anhand der Zunahme von Abbauprodukten (=Verunreinigungen).

**Tabelle 3 Stabilitätstests**

| *Stabilitätstests 25 °C*/*60 % r. h.* | **Lagerzeit** | | | |
|---|---|---|---|---|
| | **0 Monate** | **6 Monate** | **0 Monate** | **6 Monate** |
| *Lagerungssiabilitätstests* | Esmolol Formulierung ohne Cyclodextrin (Beispiel 4) | | Esmolol Formulierung mit Cyclodextrin (Beispiel 3a) | |
| **Verunreinigungen** | | | | |
| VERUNREINIGUNG A | 0 | 2.56 ± 0.53 | 0 | 1.94 ± 0.06 |
| VERUNREINIGUNG B | n.d. | n.d. | n.d. | n.d. |
| VERUNREINIGUNG C | n.d. | n.d. | n.d. | n.d. |
| VERUNREINIGUNG D | n.d. | 0.18±0.01 | n.d. | n.d. |
| Unbekannte Verunreinigungen | 0 | 0.37±0.04 | 0 | 0.38±0.02 |
| Gesamt | 0 | 3.11 | 0 | 2.24 |

### BEISPIEL 7:

### Bestimmung der Osmolarität

Die Osmolarität bzw. Gefrierpunktserniedrigung gegenüber Wasser wurde mit einem Knauer Semi-Mikro-Osmometer bestimmt. Um die Osmolarität mit diesem Osmometer bestimmen zu können, werden die Proben im Osmometer bis zum Gefrieren abgekühlt.

Die 5% Esmolol Lösung mit α-Cylodextrin gemäß Beispiel 3a weist eine Osmolarität von 290 mosmol/l auf. Dies entspricht einer isotonen Lösung, da der Bereich der Isotonie von 281 bis 297 mosmol / 1 reicht. Lösungen > 310 mosmol / 1 würde man als hyperton bezeichnen, Lösungen < 270 mosmol / 1 werden als hypoton eingestuft.

### BEISPIEL 8:

### Herstellung und Stabilitätstests von Cyclodextrin-Landiolol-Komplexen

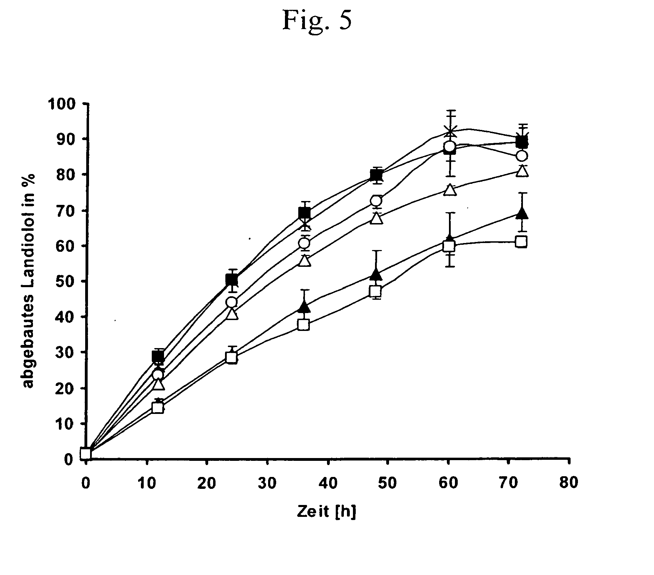

Landiolol wurde in gereinigtem Wasser in einer Konzentration von 0,25 % (m/v) gelöst. Anschließend wurde α-Cyclodextrin (Fa. Cyclolab, Budapest), 2-hydroxypropyl-β-Cyclodextrin (Fa. CTD Inc, Florida) und γ-Cyclodextrin (Fa. ISP, Deutschland) in den Endkonzentrationen 0%, 0,5%, 1%, 2% und 7% (w/v) zugegeben. Die Lösungen wurden auf 70°C erhitzt und die Stabilität von Landiolol gemäß der in Beispiel 5 beschriebenen HPLC Methode bestimmt. Die Detektion von Landiolol erfolgte dabei bei 220 nm. Die Retentionszeit von Landiolol-Hydrochlorid betrug durchschnittlich 10,5 Minuten, die des Hauptabbauproduktes 1,4 Minuten. Zur Bestimmung des Abbaus wurde das Verhältnis des Hauptabbauproduktes zum verbleibenden Landiolol Hydrochlorid berechnet und in Prozent angegeben ("Landiolol abgebaut (%)"). Die Ergebnisse dieser Studie sind in Figur 5-7 gezeigt. Die jeweils gezeigten Werte sind Mittelwerte aus 3 Versuchen ± S.D. Figur 5 zeigt den Einfluß von 0% (■), 0.5% (X), 1% (O), 2% (Δ), 4% (▲) und 7% (□) α-Cyclodextrin auf die Stabilität von Landiolol bei 70°C.

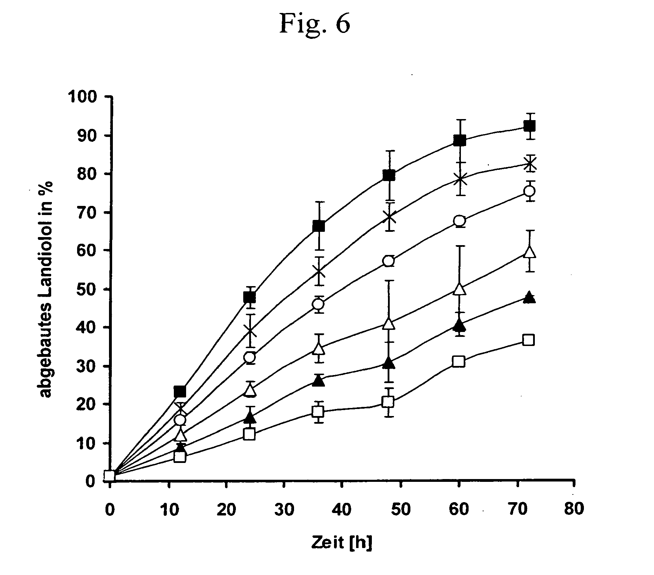

Figur 6 zeigt den Einfluß von 0% (■), 0.5% (X), 1% (O), 2% (Δ), 4% (▲) und 7% (□) 2-Hydroxypropyl-β-Cyclodextrin auf die Stabilität von Landiolol bei 70°C.

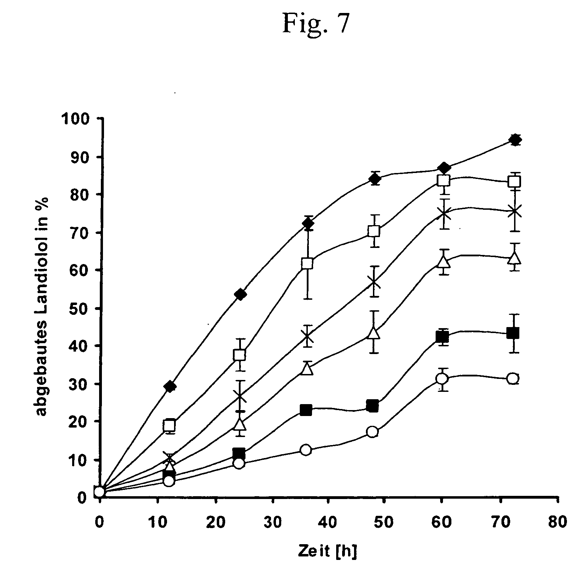

Figur 7 zeigt den Einfluß von 0% (◆), 0.5% (□), 1% (X), 2% (Δ), 4% (■) und 7% (O) γ-Cyclodextrin auf die Stabilität von Landiolol bei 70°C.

### BEISPIEL 9:

### Evaluierung des Einflusses des pH Wertes auf die Stabilität von Landiolol

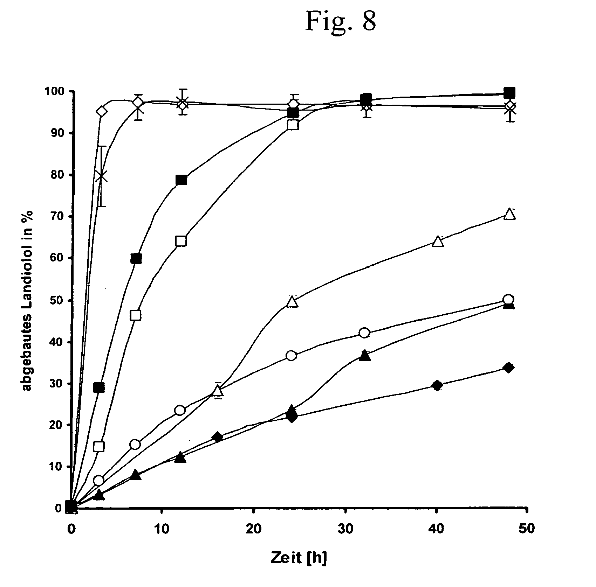

Landiolol wurde in gereinigtem Wasser in einer Konzentration von 0,25 % (w/v) gelöst. Anschließend wurde der pH-Wert auf 3; 4; 5; 5,5; 6; 6,5; 7 und 8 eingestellt. Die Lösungen wurden auf 70°C erhitzt und die Stabilität von Landiolol gemäß der in Beispiel 5 und 8 beschriebenen HPLC Methode bestimmt. Die Ergebnisse dieser Studie sind in Figur 8 gezeigt. Dabei wird der Abbau von Landiolol bei pH 3.0 (◊), pH 4.0 (X), pH 5.0 (□), pH 5.5 (Δ), pH 6.0 (▲), pH 6.5 (◆), pH 7.0 (O) und pH 8.0 (■) gezeigt. Die jeweils gezeigten Werte sind Mittelwerte aus 3 Versuchten ± S.D.

### BEISPIEL 10:

### Stabilitätstests von Cyclodextrin-Landiolol-Komplexen, die mittels konzentrierter Suspensionen hergestellt wurden

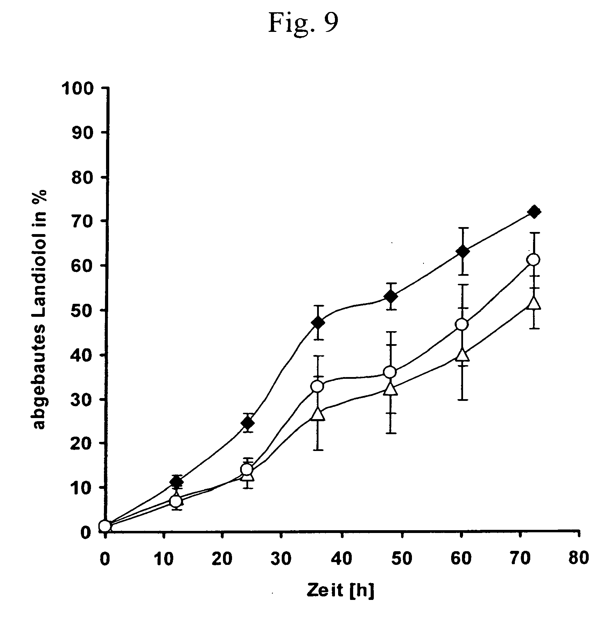

Landiolol und α-Cyclodextrin (Fa. Cyclolab, Budapest), 2-hydroxypropyl-β-Cyclodextrin (Fa. CTD Inc, Florida) bzw. γ-Cyclodextrin (Fa. ISP, Deutschland) wurde in gereinigtem Wasser in einer Konzentration von 10% Landiolol (w/v) bzw. 80% Cyclodextrin (w/v) suspendiert und zwei Stunden bei Raumtemperatur gerührt. Nach 5 minütiger Behandlung mit Ultraschall werden die Suspensionen schrittweise so verdünnt, dass die Endkonzentration von Landiolol 0,25% (w/v) beträgt. Diese Lösungen werden bei 70°C inkubiert und die gezogenen Proben mittels der in Besipiel 5 und 8 beschriebenen HPLC-Methode analysiert. Die Ergebnisse dieser Studie werden in Figur 9 dargestellt. Dabei wird der Einfluß von 2% α-Cyclodextrin (◆), 2% 2-hydroxypropyl-β-Cyclodextrin (Δ) und 2% γ-Cyclodextrin (O) auf die Stabilität von Landiolol bei 70°C gezeigt. Die jeweils gezeigten Werte sind Mittelwerte aus 3 Versuchten ± S.D.

### BEISPIEL 11:

### Gefäßschonende Wirkung von Cycodextrin auf intravenös verabreichte ultrakurzwirksame ß-Adrenorezeptor Antagonisten

Lösungen der genannten β-Adrenoreceptor Antagonisten mit oder ohne Cyclodextrin werden in Ratten chronisch über die Jugular-Vene für längere Zeit infundiert. Es zeigt sich, dass die genannten Beta-Adrenorezeptor-Antagonisten in Cyclodextrin-hältigen Lösungen deutlich weniger Endothel- und Gefäßschäden hervorrufen als bei Verwendung einer herkömmlichen Lösung.

## Patentansprüche

1. Verwendung eines Cyclodextrins und/oder eines funktionellen Cyclodextrin-Derivates ausgewählt aus der Gruppe bestehend aus α-Cyclodextrin, β-Cyclodextrin, γ-Cyclodextrin, (2-Hydroxypropyl)-β-cyclodextrin und (Sulfobutylether)-7β-cyclodextrin und Mischungen daraus, zur Erhöhung der Stabilität eines ultrakurzwirksamen β-Adrenorezeptor Antagonisten, ausgewählt aus der Gruppe bestehend aus Esmolol, Landiolol, Flestolol oder einem pharmazeutisch akzeptablen Salz davon in einer zur parenteralen Verabreichung geeigneten lagerstabilen wässerigen Lösung.

2. Pharmazeutische Zusammensetzung zur parenteralen Verabreichung eines ultrakurzwirksamen β-Adrenorezeptor Antagonisten in Form einer lagerstabilen Lösung, im wesentlichen bestehend aus
a) einem ultrakurzwirksamen β-Adrenorezeptor Antagonisten, ausgewählt aus der Gruppe bestehend aus Esmolol, Landiolol, Flestolol oder einem pharmazeutisch akzeptablen Salz davon
b) Wasser
c) einem Cyclodextrin und/oder einem funktionellen Cyclodextrin-Derivat ausgewählt aus der Gruppe bestehend aus α-Cyclodextrin, β-Cyclodextrin, γ-Cyclodextrin, (2-Hydroxypropyl)-β-cyclodextrin und (Sulfobutylether)-7β-cyclodextrin.

3. Pharmazeutische Zusammensetzung nach Anspruch 2 enthaltend weitere Hilfsstoffe ausgewählt aus Puffer, Konservierungsmittel, mit Wasser mischbare Lösungsmittel, Salze, Zuckeralkohole und/oder Zucker.

4. Verwendung oder Zusammensetzung gemäß einem der Ansprüche 1 bis 3 **dadurch gekennzeichnet, dass** der pH-Wert der Lösung 3 bis 7,5, bevorzugt 5 bis 7 beträgt.

5. Verwendung oder Zusammensetzung gemäß einem der Ansprüche 1 bis 4, **dadurch gekennzeichnet, dass** die Konzentration des Cyclodextrins bzw. des funktionellen Derivates davon 0,1% bis 20% (w/v), bevorzugt 0,25% bis 7% (w/v), besonders bevorzugt 0,5% bis 4% beträgt.

6. Verwendung oder Zusammensetzung gemäß einem der vorhergehenden Ansprüche, **dadurch gekennzeichnet, dass** die Konzentration des ultrakurzwirksamen β-Adrenorezeptor Antagonisten bzw. des Salzes davon 0,1% bis 30% beträgt.

7. Verwendung oder Zusammensetzung gemäß einem der vorhergehenden Ansprüche, **dadurch gekennzeichnet, dass** die Lösung eine Osmolarität von 270 mosmol/l bis 310 mosmol/l, bevorzugt 280 mosmol/l bis 300 mosmol/l aufweist.

8. Verwendung oder Zusammensetzung gemäß einem der vorhergehenden Ansprüche, wobei die Zusammensetzung in Form einer Verkaufseinheit ausgewählt aus der Gruppe bestehend aus - 1 ml Lösung enthaltend 10-20 mg Esmolol oder ein Salz davon - 2 ml Lösung enthaltend 10-100 mg Esmolol oder ein Salz davon - 5 ml Lösung enthaltend 50-500 mg Esmolol oder ein Salz davon - 10 ml Lösung enthaltend 50-5000 mg, insbesondere 2500 mg Esmolol oder ein Salz davon - 50 ml Lösung enthaltend 50-5000 Esmolol oder ein Salz davon - 100 ml Lösung enthaltend 50-5000 mg Esmolol oder ein Salz davon - 250 ml Lösung enthaltend 50-5000 mg Esmolol oder ein Salz davon vorliegt.

9. Verwendung oder Zusammensetzung gemäß einem der Ansprüche 1 bis 8, wobei die Zusammensetzung in Form einer Verkaufseinheit ausgewählt aus der Gruppe bestehend aus - 1 ml Lösung enthaltend 5-20 mg Landiolol oder Flestolol oder ein Salz davon - 2 ml Lösung enthaltend 5-100 mg Landiolol oder Flestolol oder ein Salz davon - 5 ml Lösung enthaltend 10-500 mg Landiolol oder Flestolol oder ein Salz davon - 10 ml Lösung enthaltend 10-5000 mg Landiolol oder Flestolol oder ein Salz davon - 25 ml Lösung enthaltend 25-2500 mg Landiolol oder Flestolol oder ein Salz davon - 50 ml Lösung enthaltend 50-5000 mg Landiolol oder Flestolol oder ein Salz davon - 100 ml Lösung enthaltend 50-5000 mg Landiolol oder Flestolol oder ein Salz davon - 250 ml Lösung enthaltend 50-5000mg Landiolol oder Flestolol oder ein Salz davon vorliegt.

10. Verwendung einer Zusammensetzung gemäß einem der vorhergehenden Ansprüche zur Herstellung eines Medikamentes zur Senkung der Ventrikelfrequenz bei Patienten mit Vorhofflimmern, Vorhofflattern und bei Sinustachykardie, bei atrioventrikulärer und bei AV-Knoten-Tachykardie, bei tachykarden supra- und ventrikulären Arrhythmien, bei Tachykardie und/oder Bluthochdruck vor, während und nach Operationen sowie in anderen Notfällen, zur Prophylaxe und Therapie perioperativer Ischämien, zur Therapie instabiler Angina pectoris und des akuten Myokardinfarkts.

## Claims

1. Use of a cyclodextrin and/or a functional cyclodextrin derivative selected from the group consisting of α-cyclodextrin, β-cyclodextrin, γ-cyclodextrin, (2-hydroxypropyl)-β-cyclodextrin and (sulfobutylether)-7β-cyclodextrin and mixtures thereof, for increasing the stability of an ultrashort-effective β-adrenoreceptor antagonist selected from the group consisting of esmolol, landiolol, flestolol or a pharmaceutically acceptable salt thereof, in a storage-stable aqueous solution suitable for parenteral administration.

2. A pharmaceutical composition for parenteral administration of an ultrashort-effective β-adrenoreceptor antagonist in the form of a storage-stable solution, substantially consisting of
a) an ultrashort-effective β-adrenoreceptor antagonist selected from the group consisting of esmolol, landiolol, flestolol or a pharmaceutically acceptable salt thereof,
b) water,
c) a cyclodextrin and/or a functional cyclodextrin derivative selected from the group consisting of α-cyclodextrin, β-cyclodextrin, γ-cyclodextrin, (2-hydroxypropyl)-β-cyclodextrin and (sulfobutylether)-7β-cyclodextrin.

3. The pharmaceutical composition according to claim 2, comprising further excipients selected from buffer, preservative, solvents mixable with water, salts, sugar alcohols and/or sugars.

4. The use or composition according to any of claims 1 to 3, **characterized in that** the pH value of said solution is 3 to 7.5, preferably 5 to 7.

5. The use or composition according to any of claims 1 to 4, **characterized in that** the concentration of said cyclodextrin or said functional derivative thereof, respectively, is 0.1 % to 20 % (w/v), preferably 0.25 % to 7 % (w/v), particularly preferred 0.5 % to 4 %.

6. The use or composition according to any of the preceding claims, **characterized in that** said concentration of said ultrashort-effective β-adrenoreceptor antagonist or said salt thereof, respectively, is 0.1 % to 30 %.

7. The use or composition according to any of the preceding claims, **characterized in that** said solution has an osmolarity of 270 mosmol/l to 310 mosmol/l, preferably 280 mosmol/l to 300 mosmol/l.

8. The use or composition according to any of the preceding claims, wherein said composition is present in the form of a sales unit selected from the group consisting of- 1 ml of solution comprising 10-20 mg of esmolol or a salt thereof - 2 ml of solution comprising 10-100 mg of esmolol or a salt thereof - 5 ml of solution comprising 50-500 mg of esmolol or a salt thereof - 10 ml of solution comprising 50-5000 mg, in particular 2500 mg of esmolol or a salt thereof - 50 ml of solution comprising 50-5000 mg of esmolol or a salt thereof - 100 ml of solution comprising 50-5000 mg of esmolol or a salt thereof - 250 ml of solution comprising 50-5000 mg of esmolol or a salt thereof.

9. The use or composition according to any of claims 1 to 8, wherein said composition is present in the form of a sales unit selected from the group consisting of- 1 ml of Solution comprising 5-20 mg of landialol or flestolol or a salt thereof - 2 ml of solution comprising 5-100 mg of landiolol or flestolol or a salt thereof- 5 ml of solution comprising 10-500 mg of landiolol or flestolol or a salt thereof - 10 ml of solution comprising 10-5000 mg of landiolol or flestolol or a salt thereof - 25 ml of solution comprising 25-2500 mg of landiolol or flestolol or a salt thereof- 50 ml of solution comprising 50-5000 mg of landiolol or flestolol or a salt thereof - 100 ml of solution comprising 50-5000 mg of landiolol or flestolol or a salt thereof - 250 ml of solution comprising 50-5000 mg of landiolol or flestolol or a salt thereof.

10. The use of a composition according to any of the preceding claims for manufacturing a drug for lowering the ventricle frequency in patients with atrial fibrillation, atrial flutter and with sinus tachycardia, with atrioventricular and with AV node tachycardia, with tachycardiac supra and ventricular arrhythmias, with tachycardia and/or hypertension prior to, during and after surgeries as well as in other emergencies, for prophylaxis and therapy of perioperative ischemias, for therapy of instable angina pectoris and acute myocardial infarction.

## Revendications

1. Utilisation d'une cyclodextrine et/ou d'un dérivé fonctionnel de cyclodextrine choisis dans le groupe constitué d'α-cyclodextrine, de β-cyclodextrine, de γ-cyclodextrine, de (2-hydroxypropyl)-β-cyclodextrine et de (sulfobutyléther)-7β-cyclodextrine et de leurs mélanges, pour augmenter la stabilité d'un antagoniste ayant une action ultra-courte sur des récepteurs β-adrénergiques, choisi dans le groupe constitué d'esmolol, de landiolol, de flestolol ou de l'un de leurs sels pharmaceutiquement acceptables, dans une solution aqueuse stable au stockage et apte à une administration parentérale.

2. Composition pharmaceutique destinée à l'administration parentérale d'un antagoniste ayant une action ultra-courte sur des récepteurs β-adrénergiques, sous forme d'une solution stable au stockage constituée essentiellement
a) d'un antagoniste ayant une action ultra-courte sur des récepteurs β-adrénergiques, choisi dans le groupe constitué d'esmolol, de landiolol, de flestolol ou de l'un de leurs sels pharmaceutiquement acceptables
b) d'eau
c) d'une cyclodextrine et/ou d'un dérivé fonctionnel de cyclodextrine choisis dans le groupe constitué d'α-cyclodextrine, de β-cyclodextrine, de γ-cyclodextrine, de (2-hydroxypropyl)-β-cyclodextrine et de (sulfobutyléther)-7β-cyclodextrine.

3. Composition pharmaceutique selon la revendication 2, contenant en outre des agents auxiliaires choisis parmi les tampons, les agents conservateurs, les solvants miscibles à l'eau, les sels, les polyols et/ou les sucres.

4. Utilisation ou composition selon l'une des revendications 1 à 3, **caractérisée en ce que** le pH de ladite solution est compris entre 3 et 7,5, de préférence entre 5 et 7.

5. Utilisation ou composition selon l'une des revendications 1 à 4, **caractérisée en ce que** la concentration de la cyclodextrine et/ou de son dérivé fonctionnel est comprise entre 0,1 % et 20 % w/v, de préférence entre 0,25 % et 7 % w/v, avec une préférence particulière entre 0,5 % et 4 %.

6. Utilisation ou composition selon l'une des revendications précédentes, **caractérisée en ce que** la concentration dudit antagoniste ayant une action ultra-courte sur des récepteurs β-adrénergiques ou de son sel est comprise entre 0,1 % et 30 %.

7. Utilisation ou composition selon l'une des revendications précédentes, **caractérisée en ce que** ladite solution présente une osmolarité comprise entre 270 mosmol/l et 310 mosmol/l, de préférence entre 280 mosmol/l et 300 mosmol/l.

8. Utilisation ou composition selon l'une des revendications précédentes, ladite composition se présentant sous forme d'une unité de vente choisie dans le groupe constitué de - 1 ml de solution contenant 10-20 mg d'esmolol ou d'un de ses sels - 2 ml de solution contenant 10-100 mg d'esmolol ou d'un de ses sels - 5 ml de solution contenant 50-500 mg d'esmolol ou d'un de ses sels - 10 ml de solution contenant 50-5000 mg, notamment 2500 mg d'esmolol ou d'un de ses sels - 50 ml de solution contenant 50-5000 mg d'esmolol ou d'un de ses sels - 100 ml de solution contenant 50-5000 mg d'esmolol ou d'un de ses sels - 250 ml de solution contenant 50-5000 mg d'esmolol ou d'un de ses sels.

9. Utilisation ou composition selon l'une des revendications 1 à 8, ladite composition se présentant sous forme d'une unité de vente choisie dans le groupe constitué de - 1 ml de solution contenant 5-20 mg de landiolol ou de flestolol ou d'un de leurs sels - 2 ml de solution contenant 5-100 mg de landiolol ou de flestolol ou d'un de leurs sels - 5 ml de solution contenant 10-500 mg de landiolol ou de flestolol ou d'un de leurs sels - 10 ml de solution contenant 10-5000 mg de landiolol ou de flestolol ou d'un de leurs sels - 25 ml de solution contenant 25-2500 mg de landiolol ou de flestolol ou d'un de leurs sel- 50 ml de solution contenant 50-5000 mg de landiolol ou de flestolol ou d'un de leurs sels - 100 ml de solution contenant 50-5000 mg de landiolol ou de flestolol ou d'un de leurs sels - 250 ml de solution contenant 50-5000 mg de landiolol ou de flestolol ou d'un de leurs sels.

10. Utilisation d'une composition selon l'une des revendications précédentes pour préparer un médicament destiné à baisser la fréquence ventriculaire chez des patients atteints d'une fibrillation atriale, d'un flutter atrial, ainsi que dans le cas d'une tachycardie sinusale, d'une tachycardie atrio-ventriculaire et du noeud atrio-ventriculaire, d'arythmies se manifestant par une tachycardie supra- et ventriculaire, d'une tachycardie et/ou d'une hypertension survenant avant, durant ou après des opérations et dans d'autres cas d'urgence, à la prévention et la thérapie d'ischémies péri-opératoires, à la thérapie d'une angine de poitrine instable et de l'infarctus du myocarde aigu.
